(19) Europäisches Patentamt
European Patent Office
Office européen des brevets

(11) EP 1 491 627 B1

(12) # EUROPEAN PATENT SPECIFICATION

(45) Date of publication and mention of the grant of the patent:
**27.09.2006 Bulletin 2006/39**

(21) Application number: **03710428.8**

(22) Date of filing: **19.03.2003**

(51) Int Cl.:
***C12N 15/09*** *(2006.01)* ***C12Q 1/68*** *(2006.01)*
***G01N 33/53*** *(2006.01)* ***G01N 33/566*** *(2006.01)*

(86) International application number:
**PCT/JP2003/003307**

(87) International publication number:
**WO 2003/083108 (09.10.2003 Gazette 2003/41)**

(54) **GENE EXAMINATION METHOD FOR JUDGING THE ONSET RISK OF GLAUCOMA**

GENUNTERSUCHUNGSVERFAHREN ZUR BEURTEILUNG DES RISIKOS FÜR DAS AUFTRETEN VON GLAUKOM

TECHNIQUE D'EXAMEN DE GENE PERMETTANT D'ESTIMER LE RISQUE DE SURVENUE DE GLAUCOME

(84) Designated Contracting States:
**AT BE BG CH CY CZ DE DK EE ES FI FR GB GR HU IE IT LI LU MC NL PT RO SE SI SK TR**

(30) Priority: **29.03.2002 JP 2002093443**

(43) Date of publication of application:
**29.12.2004 Bulletin 2004/53**

(73) Proprietor: **SYSMEX CORPORATION**
**Kobe-shi,**
**Hyogo 651-0073 (JP)**

(72) Inventors:
- **ASANO, Kaoru,**
**Sysmex Corporations**
**Kobe-shi,**
**Hyogo 651-0073 (JP)**
- **TAKAHATA, Takayuki,**
**Sysmex Corporation**
**Kobe-shi,**
**Hyogo 651-0073 (JP)**
- **NUMADA, Shigehiro,**
**Sysmex Corporation**
**Kobe-shi,**
**Hyogo 651-0073 (JP)**
- **MASAGO, Akinori,**
**Sysmex Corporation**
**Kobe-shi,**
**Hyogo 651-0073 (JP)**
- **KOUCHI, Yasuhiro,**
**Sysmex Corporation**
**Kobe-shi,**
**Hyogo 651-0073 (JP)**

(74) Representative: **De Clercq, Ann G. Y. et al**
**De Clercq, Brants & Partners c.v.,**
**Edgard Gevaertdreef 10a**
**9830 Sint-Martens-Latem (BE)**

(56) References cited:
**WO-A-00/42220** **WO-A-99/16898**
**WO-A-99/51779**

- **DATABASE EMBL [Online] 19 December 2000 (2000-12-19), "1M0443E12R Mouse 10kb plasmid UUGC1M library Mus musculus genomic clone UUGC1M0443E12 R, DNA sequence." XP002319999 retrieved from EBI accession no. EM_PRO:AZ614698 Database accession no. AZ614698**
- **DATABASE EMBL [Online] 10 May 2001 (2001-05-10), "Sequence 1259 from Patent WO0129262." XP002320000 retrieved from EBI accession no. EM_PRO:AX116136 Database accession no. AX116136**
- **DATABASE EMBL [Online] 21 December 2002 (2002-12-21), "Sequence 24 from patent US 6475724." XP002320001 retrieved from EBI accession no. EM_PRO:AR242767 Database accession no. AR242767**
- **DATABASE Geneseq [Online] 7 February 2000 (2000-02-07), "Human GLC1A gene exon 1 specific forward primer." XP002320002 retrieved from EBI accession no. GSN:AAZ37981 Database accession no. AAZ37981**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 491 627 B1

- DATABASE Geneseq [Online] 21 November 1996 (1996-11-21), "OVCA1 gene exon 13c 5' primer." XP002320003 retrieved from EBI accession no. GSN:AAT36952 Database accession no. AAT36952
- DATABASE EMBL [Online] 23 February 2001 (2001-02-23), "2M0110M16R Mouse 10kb plasmid UUGC1M library Mus musculus genomic clone UUGC2M0110M16 R, DNA sequence." XP002320004 retrieved from EBI accession no. EM_PRO:AZ831199 Database accession no. AZ831199
- DATABASE Geneseq [Online] 12 September 2001 (2001-09-12), "Porcine Gal alpha(1,3) galactosyl transferase intron 4, PCR primer #2." XP002320005 retrieved from EBI accession no. GSN:AAS02341 Database accession no. AAS02341
- DATABASE EMBL [Online] 23 February 2001 (2001-02-23), "2M0093O23R Mouse 10kb plasmid UUGC1M library Mus musculus genomic clone UUGC2M0093O23 R, DNA sequence." XP002320006 retrieved from EBI accession no. EM_PRO:AZ821264 Database accession no. AZ821264
- DATABASE EMBL [Online] 8 April 1996 (1996-04-08), "Sequence 5 from patent US 5484909." XP002320007 retrieved from EBI accession no. EM_PRO:I17219 Database accession no. I17219
- DATABASE EMBL [Online] 7 November 2001 (2001-11-07), "Ciona intestinalis cDNA, clone: rcilv05i16, 3' end, single read." XP002320008 retrieved from EBI accession no. EM_PRO: AV851788 Database accession no. AV851788
- DATABASE Geneseq [Online] 6 April 2000 (2000-04-06), "PCR primer A used to amplify a fragment of G3BP polynucleotide." XP002320009 retrieved from EBI accession no. GSN:AAZ45443 Database accession no. AAZ45443
- DATABASE EMBL [Online] 6 October 1999 (1999-10-06), "Sequence 416 from patent US 5851760." XP002320010 retrieved from EBI accession no. EM_PRO:AR067068 Database accession no. AR067068
- DATABASE Geneseq [Online] 10 September 2001 (2001-09-10), "Human biallelic marker upstream amplification primer SEQ ID NO:6705." XP002320011 retrieved from EBI accession no. GSN:AAZ72349 Database accession no. AAZ72349
- DATABASE EMBL [Online] 22 August 1997 (1997-08-22), "Human DNA sequence from clone RP3-454G6 on chromosome 1q24 Contains the 3' end of the gene for HBxAg transactivated protein 2 (XTP2) and the MYOC gene for myocilin, trabecular meshwork inducible glucocorticoid response" XP002320012 retrieved from EBI accession no. EM_PRO:HS454G6 Database accession no. HS454G6
- COBB C.J. ET AL. BR. J. OPHTHALMOL. vol. 86, February 2002, pages 191 - 195, XP002968830
- MABUCHI F. ET AL. CLIN. GENET. vol. 59, 2001, pages 263 - 268, XP002968831
- KUBOTA R. ET AL. HUMAN MUTATION vol. 16, no. 3, 2000, page 270, XP002968832
- SHIMIZU S. ET AL. AM. J. OPHTHALMOL. vol. 130, no. 2, 2000, pages 165 - 177, XP002968833
- FINGERT J.H. ET AL. HUMAN MOL. GENET. vol. 8, no. 5, 1999, pages 899 - 905, XP002968834
- ALLINGHAM R.R. ET AL. INVEST. OPHTHALMOL. VIS. SCI. vol. 139, 1998, pages 2288 - 2295, XP002968835
- ROZSA F.W. ET AL. MOLECULAR VISION vol. 4, no. 20, 1998, pages 1 - 16, XP002968836

## Description

FIELD OF THE INVENTION

The present invention relates to a method for examining a glaucoma-related gene in the field of a clinical examination, and an examination method employing a variation in this gene as an index for the purpose of predicting any risk of developing glaucoma. For example, a method for examining a gene by detecting any abnormality in a myocilin (hereinafter referred to as MYOC) gene known as a glaucoma gene followed by diagnosing the glaucoma using as an index the abnormality thus detected, i.e., a variation in a base in a particular site in the gene, especially, an examination method for predicting any possibility of developing in future for a certain individual, is contemplated.

BACKGROUND OF THE INVENTION

Glaucoma is a disease involving a difficulty in excreting an aqueous humor in an eye, which leads to an increased ocular tension, resulting in a reduced ocular function. If being left untreated, it reduces the visual field and the sight, resulting in a blindness. A normal ocular tension, however, may also cause an impairment of an optic nerve.

Glaucoma is classified into any one of the five pathological types, namely, primary open-angle glaucoma (POAG), normal tension glaucoma (NTG), primary angle-closure glaucoma (PACG), congenital glaucoma and secondary glaucoma, and 20% of the glaucoma is considered to be congenital. Among these types, the most frequent one is POAG. The epidemiological investigation conducted over the period from 1988 to 1989 by Japan Ophthalmologists Association, reported that 3.56% of the population of 40 years old or older consists of the glaucoma patient.

A major risk factor of glaucoma is a familial history, and the development of glaucoma is suggested positively to be associated with a gene. In the United States Patent No.5,789,169 to Nguyen et al filed on May 17, 1996, a gene encoding a TIGR (trabecular reticulum-induced glucocorticoid responsive) protein is disclosed as a glaucoma-related gene. The TIGR gene is referred to also as MYOC gene. The United States Patent No. 5,789,169 to Nguyen et al also disclosed the cDNA sequence of this protein, the protein itself, a molecule binding thereto and a nucleic acid molecule encoding this binding molecule, and provided improved methods and reagents for diagnosing glaucoma and related diseases, as well as for diagnosing cardiovascular diseases, immune diseases or other diseases or conditions affecting the expression or activity of this protein. On the other hand, a method for diagnosing glaucoma in an individual by detecting a mutation in a CYP1B1 gene among the glaucoma-related genes followed by using the presence of this mutation as an index of glaucoma is disclosed (JP-W-2001-512969). Nevertheless, any of the publications described above was not successful in disclosing a means for predicting any risk of developing glaucoma in future, although it focused on the relationship between the glaucoma-related genes and the glaucoma.

On the other hand, WO01/88120A1 discloses a method for detecting a variation in the gene at position -153 in an MYOC gene promoter region represented in the sequence listing in this publication, and describes that the method can be used in screening for glaucoma in a patient whose familial genetic predisposition is problematic or who is suspected to be a carrier not developed. Nevertheless, the variation just at position -153 as the only one position is just focused on here and used as an index.

WO 99/16898 discloses that two novel mutations (single-base substitutions) in the coding region of the MYOC gene can cause glaucoma. WO 99/16898 provides a method to detect these two mutations as a means for early identification of subjects at risk for developing glaucoma. WO 99/16898 does not disclose detection of sequence variations in the upstream region of the MYOC gene for predicting glaucoma.

WO 00/42220 discloses a -5300-bp region upstream of the coding sequence of the MYOC gene and further discloses six variations in this upstream region. These mutations, which include single-base substitutions and a 4-bp insertion, are found in subjects with glaucoma, but not in non-glaucomic subjects. Accordingly, WO 00/42220 provides a method for diagnosing and prognosing of glaucoma comprising detection of these polymorphisms. WO 00/42220 does not suggest combined detection of variations from the coding and upstream regions of the MYOC gene to predict glaucoma.

Mabuchi et al. (Clin Genet, Vol. 59, No. 4, pages 263-268, of April 2001) genotyped the MYOC gene in 114 unrelated Japanese patients with normal-tension glaucoma (NTG), 119 patients with POAG and 100 control subjects without glaucoma. They identified 5 amino acid sequence changes in MYOC: Arg46Stop (one NTG), Arg76Lys (four NTG, 10 POAG, seven control), Arg158Gln (one NTG, one POAG, one control) found in only Japanese, Asp208Glu (four NTG, three POAG, one control), Pro481Ser (one control). Mabuchi et al. do not suggest combined detection of variations from the coding and upstream regions of the MYOC gene to improve prediction of glaucoma.

Kubota et al. (Hum Mutat, Vol. 16, No. 3, page 270, of September 2000) investigated the MYOC mutation prevalence in Japan in 140 unrelated Japanese patients. They identified 10 sequence variants, of which four were highly probable for disease-causing mutations (Arg46ter, Arg158Gln, Ile360Asn, and Ala363Thr), and six polymorphisms (Gln19His, Arg76Lys, Asp208Glu, Val439Val, Arg470His, and Ala488Ala). Kubota et al. did not disclose detection of sequence variations in the upstream region of the MYOC gene for predicting glaucoma.

Since glaucoma is a latent disease, a further excellent method for enabling an early diagnosis or an effective prediction of possibility for developing glaucoma is desired in order to ensure the prevention or amelioration prior to a serious impairment of an optic nerve.

DESCRIPTION OF THE INVENTION

(Problem to be Solved)

An early diagnosis and early treatment of glaucoma is possible efficiently if it is possible to identify an individual who has a genetic risk factor of the glaucoma and who is at an elevated risk of developing in future and to perform a glaucoma examination in this individual intensively. Under such a circumstance, an objective of the invention is to provide a method for examining a gene for the purpose of effectively predicting a risk of developing glaucoma based on the relationship between a glaucoma-related gene and the development of glaucoma.

(Means for Solving Problem)

We focused on the involvement of a gene variation in a development of glaucoma and analyzed the gene sequence in the upstream region and the coding region of the glaucoma-responsible gene in glaucoma patients and non-patients, and finally discovered as a result of keen examination that there is a genetic polymorphism in this gene whose frequency is different between the patient group and the non-patient group. Furthermore, we discovered that the glaucoma incidence differs in a statistically significant manner when compared with the incidence in an ordinary population on the basis of the presence or absence of this genetic polymorphism and completed the present invention.

Thus, the invention consists of:

1. A method for examining a glaucoma related myocilin (MYOC) gene comprising the steps:

   (i) detecting a first variation selected from the group consisting of, in SEQ ID No:1, a variation at position 4037 and a variation at position 4346;

   (ii) detecting a second variation selected from the group consisting of, in SEQ ID No:1, a variation at position 194, a variation at position 1051, a variation at position 1084, a variation at position 1627, a variation at position 1685, a variation at position 1756 and a variation at position 1853; and

   (iii) determining high risk of development of glaucoma based on the detection of the first and the second variations.

2. the method according to the above mentioned 1, wherein the said variation at position 4037 is a G-to-A substitution.

3. the method according to the above mentioned 1 or 2, wherein the said variation at position 4346 is a G-to-A substitution.

4. the method according to the above mentioned 1to 3, wherein the said variation at position 194 is a C-to-A substitution, the said variation at position 1051 is a C-to-T substitution, the said variation at position 1084 is a C-to-T substitution, the said variation at position 1627 is a T-to-C substitution, the said variation at position 1685 is a T-to-C substitution, the said variation at position 1756 is a C-to-T substitution, and the said variation at position 1853 is a G-to-C substitution.

5. the method according to the above mentioned 1 to 4, wherein the said glaucoma is primary open-angle glaucoma or normal tension glaucoma.

BRIEF DESCRIPTION OF THE DRAWINGS

Figure 1 shows the structure of an MYOC gene and the relative position of the primer (Example 1).
Figure 2 shows the principle of the Bayes theory (Example 2).

BEST MODE FOR CARRYING OUT THE INVENTION

We sequenced the gene sequence in an upstream region from the translation initiation point to the 4120th nucleic-acid base and also the gene sequence in the coding region following to the translation initiation point in the glaucoma-responsible gene consisting of the nucleic-acid base sequence represented by SEQ ID No: 1 for glaucoma patients and non-patients. In the course of this work, we found a genetic polymorphism in the upstream region and the coding region of this gene, whose frequency differs between a patient group and a non-patient group. In addition, we found a fact that the glaucoma incidence differs in a statistically significant manner when compared with the incidence in an ordinary population on the basis of the presence or absence of this genetic polymorphism. The present invention is constituted of new findings described-above.

(Glaucoma-related gene)

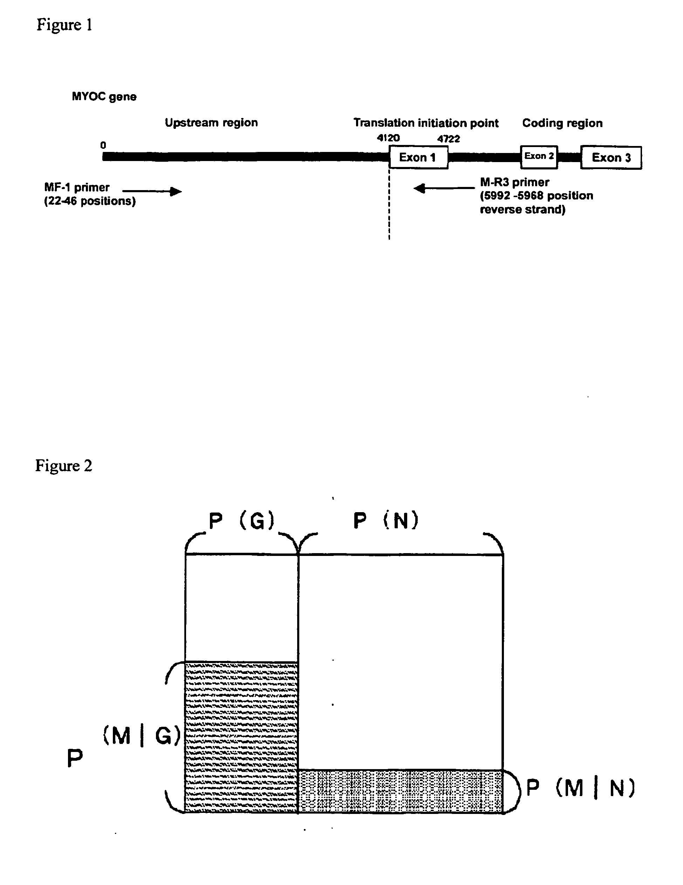

A glaucoma-related gene according to the invention is a TIGR gene (trabecular reticulum-induced glucocorticoid responsive). This TIGR gene is known also as a MYOC gene. The structures and the sequences of the upstream and coding region of the MYOC gene are as shown in Figure 1 and SEQ ID No: 1, and there are ,for example, an upstream region with a promoter element and a coding region which encodes a protein, as well as other elements. The position of nucleic-acid base of MYOC gene is in accordance with the nucleic-acid base number defined in SEQ ID No: 1 (GenBank accession number: NT_029874). The region which encodes this MYOC protein is formed from three exons. A region from position 1 to position 4120 in SEQ ID No: 1 is an upstream region, and a resion from position 4120 to position 4722-position represents Exon 1.

(Gene variation)

A variation in glaucoma-related MYOC gene is a substitution of a nucleic-acid base in a particular position in an MYOC gene with a different nucleic-acid base, as well as deletion and/or insertion. Such a particular position is subjected for example to a substitution with a different base. Such a particular position refers a position selected from the 194-position, 199-position, position 324, position 1051, position 1084, position 1627, position 1685, position 1756, position 1853, position 2830, position 3371, position 4037 and/or position 4346 in the nucleic-acid base sequence represented by SEQ ID No: 1.

An inventive typical nucleic-acid base substitution in a particular position may for example be the C-to-A substitution at position 194; the A-to-C substitution at position 199; the G-to-A substitution at position 324; the C-to-T substitution at position 1051; the C-to-T substitution at position 1084; the T-to-C substitution at position 1627; the T-to-C substitution at position 1685; the C-to-T substitution at position 1756; the G-to-C substitution at position 1853; the G-to-A substitution at position 2830; the A-to-G substitution at position 3371; the G-to-A substitution at position 4037; and the G-to-A substitution at position 4346 in the nucleic-acid base sequence represented by SEQ ID No: 1.

Preferably, at least one substitution of nucleic-acid bases at position 199; position 324; position 1051; position 1084; position 1627; position 1685; position 1756; position 1853; position 2830 and position 3371 in the nucleic-acid base sequence represented by SEQ ID No: 1 is detected and subjected to a gene examination. More preferably, at least two substitutions of nucleic-acid bases at position 194; position 199; position 324; position 1051, position 1084; position 1627; position 1685; position 1756; position 1853, position 2830; position 3371; position 4037 and position 4346 in the nucleic-acid base sequence represented by SEQ ID No: 1 is detected.

(Examination method)

The procedure in a method for examining a variation in the gene is not limited particularly as long as it can detect a particular variation of MYOC gene, and any of various methods which are known or will be available in future may be employed.

In order to examine an inventive variation in an MYOC gene in a subject, various methods for analyzing a nucleic-acid base sequence containing a position of such a variation can be employed. Such a method may for example be a southern hybridization method, dot hybridization method (see, for example, J. Mol. Biol., 98; 503-517 (1975)), dideoxy base sequencing method (Sanger method), various detection methods which combine DNA amplification technologies [for example, restriction fragment length polymorphism (RELF), PCR single strand higher structure polymorphism analysis (see, for example, Proc. Natl. Acad. Sci., U.S.A., 86:2766-2770 (1989)), PCR-specific sequence oligonucleotide method (SSO), allele-specific oligonucleotide method employing a PCR-SSO and a dot hybridization (see, for example, Nature, 324: 163-166 (1986))] and the like. As long as the position of the gene variation to be detected according to the invention is disclosed and specified, the variation can be detected using a method known in the art.

(Examination sample preparation)

In order to analyze an MYOC gene in a subject, an examination sample to be subjected to an inventive examination method is not limited particularly as long as it is a biological sample containing the MYOC gene in the subject. Such a biological sample may for example be a biological material tissue, surgically incised tissue, tissue isolated from a living body such as an oral mucosa tissue, as well as blood, serum, feces, injected semen, sputum, saliva, cerebrospinal fluid, hair and the like. An MYOC gene obtained by pelletizing a biological sample such as a tissue for example by a blender followed by extracting by a known gene extraction method such as a phenol/chloroform method can be used as an examination sample. The extracted MYOC gene may further be amplified and concentrated to give a sample.

A sample to be examined here may be a full-length DNA of an MYOC gene, or may be a DNA fragment (partial DNA). When a DNA fragment is subjected to the examination, it should contain a particular region which contains an upstream region and/or coding region of a MYOC gene and which contains a particular region involving a variation(s) in at least one position, preferably two or more position, more preferably 3 or more positions. Such a DNA fragment may not be limited particularly with regard to the base length, as long as it is one which can be utilized in detecting an inventive gene variation, i.e. one having a measurable base length of a subject DNA to be examined for the base substitution. The base length of such a DNA is usually 10 bases or more, preferably 20 bases or more, and generally, those having 100 to 1000, preferably 200 to 300 bases is selected.

A sample to be examined may be a DNA or a DNA transcription product. Typically, it may be a messenger RNA (mRNA) transcribed from a DNA, or its reverse transcription product cDNA, or a complementary DNA. Various procedures which can be employed in an inventive gene variation detecting method, including synthesis of a DNA or DNA fragment, enzyme treatment for a DNA cleavage, deletion, addition and binding, DNA isolation, purification, replication, selection as well as DNA fragment amplification, may be conducted by standard methods (see, for example, BUNSHIIDENGAKUJIKKENHO, KYORITSU SHUPPAN, published in 1983). A customary modification may also be conducted, if necessary.

The amplification of a nucleic acid for preparing a sample to be examined may be conducted for example by a PCR method or a modification thereof (see, for example, PCR Technology, Takara, published in 1990). In such a case, an oligonucleotide capable of being specifically hybridized with a part of a glaucoma-related gene, typically, an oligonucleotide possessing a primer function selected so that a desired DNA fragment having at least one particular position involved in the variation described above is amplified specifically, can be utilized.

(Primer function-possessing oligonucleotide)

A primer function-possessing oligonucleotide may for example be 1) an oligonucleotide consisting of a nucleic-acid base sequence represented by any of SEQ ID Nos. 2 to 27; 2) a strand complementary with the oligonucleotide according to the above-mentioned 1); 3) an oligonucleotide capable of hybridizing under a stringent condition with the oligonucleotide according to the above-mentioned 1) or 2); 4) an oligonucleotide having a homology of about 60% with the oligonucleotide according to any one of the above-mentioned 1) to 3); and 5) an oligonucleotide having a nucleic-acid base sequence whose 1 or more nucleic-acid base(s) of oligonucleotides according to above-mentioned 1) to 4) was subjected to a variation such as a substitution, deletion, insertion or addition.

An oligonucleotide can be designed by a method known per se, and may for example be synthesized chemically. Alternatively, a naturally occurring nucleic acid can be cleaved for example by a restriction enzyme to alter into one having the nucleic-acid base sequence described above or can be ligated. Typically, the synthesis may be effected for example by using an oligonucleotide synthesizer (Applied Biosystems, Expedite Model 8909 DNA synthesizer). A method for synthesizing an oligonucleotide involving a variation such as a substitution, deletion, insertion of addition of one to several nucleic-acid base(s) may also be a method known per se. For example, a site-specific variation introduction method, gene homologous recombination method, primer extension method or polymerase chain reaction (PCR) method can be employed alone or in appropriate combination with each other, for example, in accordance for example with the methods described in Molecular Cloning: A Laboratory Mannual, 2nd ed., Sambrook et al., Cold Spring Harbor Laboratory Press, Cold Spring Harbor, New York, 1989; LABOMANUAL GENE ENGINEERING, ed by Muramatsu, Maruzene, 1988; PCR TECHNOLOGY, PRINCIPLE and APPLICATION OF DNA AMPLIFICATION, ed by Ehrlich, H.E., Stockton Press, 1989 and the like, with or without any modification, as well as the technology by Ulmer (Science (1983), 219:666).

A stringent hybridization condition may be any condition known commonly, such as the condition involving a hybridization at 42°C overnight in a solution containing 50% formamide, 5 x SSC (150 mM NaCl, 15mM trisodium citrate), 50 mM sodium phosphate, pH7.6, 5 x Denhart's solution, 10% dextran sulfate and 20 μg/ml DNA, followed by a primary washing with 2 x SSC/0.1% SDS at room temperature, followed by a secondary washing with 0.1 x SSC/0.1% SDS at about 65°C.

(Detection of DNA variation)

A variation in a DNA can be detected for example by sequencing the MYOC gene contained in a sample to be examined by Sanger method.

An oligonucleotide complementary with a single-stranded target MYOC gene is hybridized to the target MYOC gene and used as a primer to synthesis a complementary chain in the direction of 5' to 3' using a DNA polymerase. The oligonucleotide employed here may for example be an oligonucleotide described above (a primer function-possessing oligonucleotide), which is used as a primer.

In addition to 4 types of deoxyncleotide triphosphates (dNTPs), small amounts of dideoxyncleotide triphosphates (ddNTPs) are added as reaction substrates separately for respective nucleic-acid bases and the complementary chain is synthesized. ddNTP is a dNTP analogue formed as a result of the replacement of the -OH group at 3'-position of a deoxyribose with a -H group, and the incorporation of the ddNTP instead of the dNTP prevents any further synthesis of a complementary chain, thus enabling the synthesis of DNAs having various lengths. The addition for example of a chemiluminescence- or radioisotope (RI)-labeled primer or dNTP to a reaction system enables the labeling of a DNA to be synthesized, thus enabling the nucleic-acid base sequencing by subjecting the reaction products to an electrophoresis on a modified polyacrylamide gel.

A DNA polymerase employed in the Sanger method may for example be a klenow enzyme, T7 phage and thermophilic microorganism-derived DNA polymerase and the like. Commonly from these, the exonuclease activity was removed in a gene engineering manner. Initially, the Sanger method employed an intended gene as a single-stranded DNA, but currently a double-stranded plasmid is employed frequently as being alkali-modified directly.

A sequence reaction can be conducted by the Sanger method or a cycle sequence method. The cycle sequence method involves the Sanger method in combination with a PCR, and needs no process to convert a template DNA into a single strand, and employs a DNA, one primer, dNTPs, ddNTPs and a heat resistant DNA polymerase to be added to the reaction system. During the PCR reaction, the dNTPs are incorporated to arrest the chain elongation, as a result, allowing the DNAs having identical bases at their 3'-ends to be produced similarly to the Sanger method, The sequence reaction by an automatic sequencer may for example be a dye primer method using fluorescence-labeled primer, a dye terminator method using a fluorescence-labeled ddNTP and an internal-label method whose substrate dNTP is labeled.

(Examination reagents and examination reagent kits)

The invention also encompasses an examination reagent or examination reagent kit employed in a method for examining a glaucoma gene. The examination reagent may be any reagent employed in an inventive method including as a primer for amplifying a sample to be examined, a primer for nucleic-acid base sequencing of a sample to be examined, various polymerases, base substrates, labels and the like. The examination reagent kit may be any one which employs, as a kit, at least two of various reagents employed in an inventive method.

EXAMPLES

The following Examples serve to further described the invention. However, they are not intended to restrict the invention in any way.

(Example 1: DNA analysis of MYOC gene)

(1) DNA Extraction

A blood donated from a subject to be examined was treated by a standard method and a DNA was extracted from a nucleating cell. The commercial DNA extraction kit "GenTORUKUN TM (for blood)" (Takara) was used and the DNA was extracted in accordance with the protocol attached thereto.

(2) Template DNA amplification

The resultant DNA extract was employed as a template to amplify the MYOC gene by a PCR using a PCR amplification kit, trade name "LATaq" (Applied Biosystems). The amplification primers were an M-F1 (SEQ ID No: 2) as a sense primer and an M-R3 (SEQ ID No: 3) as an antisense primer. The M-F1 consists of a nucleic-acid base sequence represented in a region from position 22 to position 46 of SEQ ID No: 1, while the M-R3 consists of the sequence complementary with the nucleic-acid base sequence represented in a region from position 5992 to position 5968. The reaction involved a heating at 94°C for 1 minutes, followed by 30 cycles, each including at 94°C for 30 seconds, at 60°C for 30 seconds and then at 72°C for 5 minutes and 30 seconds. The MYOC gene exon, translation initiation point and upstream structure, and the region amplified by the primers are in the relationship shown in Figure 1.

(3) DNA Fragment Sequencing

The DNA fragments obtained by the PCR described above were subjected to an automatic DNA sequencer ABI Prism 3100 (Applied Biosystems) for sequencing the nucleic-acid base sequence of the DNA in accordance with the protocol attached thereto. The cycle sequence reaction was conducted here using the primers shown below.

The oligonucleotides represented by the following respective SEQ ID Nos. each consisting of a nucleic-acid base sequence contained in a region based on the nucleic-acid base sequence represented by SEQ ID No: 1 or a complementary sequence thereof were used as primers.

Forward primers:

| | |
|---|---|
| M-F1 | Region from position 22 to position 46 (SEQ ID No: 2); |
| M-SF1 | Region from position 372 to position 390 (SEQ ID No: 4); |
| M-SF2 | Region from position 740 to position 759 (SEQ ID No: 5); |
| M-SF3 | Region from position 1093 to position 1110 (SEQ ID No: 6); |
| M-SF4 | Region from position 1456 to position 1475 (SEQ ID No: 7); |
| M-SF5 | Region from position 1880 to position 1817 (SEQ ID No: 8); |
| M-SF6 | Region from position 2148 to position 2165 (SEQ ID No: 9); |
| M-SF7 | Region from position 2498 to position 2516 (SEQ ID No: 10); |
| M-SF8 | Region from position 2857 to position 2875 (SEQ ID No: 11); |
| M-SF9 | Region from position 3227 to position 3246 position (SEQ ID No: 12); |
| M-SF10 | Region from position 3601 to position 3620 (SEQ ID No: 13); |
| M-SF11 | Region form 3910 to position 3927 (SEQ ID No: 14); |

Reverse primers:

| | |
|---|---|
| M-SR4 | Resion from position 4730 to position 4712 complementary sequence (SEQ ID No: 15); |
| M-SR5 | Resion from position 4337 to position 4319 complementary sequence (SEQ ID No: 16); |
| M-SR6 | Resion from position 4022 to position 4003 complementary sequence (SEQ ID No: 17); |
| M-SR7 | Resion from position 3712 to position 3695 complementary sequence (SEQ ID No: 18); |
| M-SR8 | Resion from position 3379 to position 3360 complementary sequence (SEQ ID No: 19); |
| M-SR9 | Resion from position 2950 to position 2933 complementary sequence (SEQ ID No: 20); |
| M-SR10 | Resion from position 2593 to position 2575 complementary sequence (SEQ ID No: 21); |
| M-SR11 | Resion from position 2259 to position 2241 complementary sequence (SEQ ID No: 22); |
| M-SR12 | Resion from position 1950 to position 1933 complementary sequence (SEQ ID No: 23); |
| M-SR13 | Resion from position 1556 to position 1538 complementary sequence (SEQ ID No: 24); |
| M-SR14 | Resion from position 1170 to position 1153 complementary sequence (SEQ ID No: 25); |
| M-SR15 | Region from position 824 to position 807 complementary sequence (SEQ ID No: 26); |
| M-SR16 | Resion from position 470 to position 453 complementary sequence (SEQ ID No: 27). |

Among the primers listed above, M-F1 through M-SF 11 were used for sequencing the forward strands, while M-SR4 through M-SR16 were used for sequencing the reverse strands.

(4) DNA fragment ligation and MYOC gene nucleic-acid base sequence

The sequence of the DNA fragment of individual blood donor was ligated using a Phred/Phrap software (Washington University, USA) and one nucleic-acid base sequence per blood donor was obtained.

The blood samples obtained from a control group of 67 non-patient volunteers were treated in accordance with the above-mentioned procedure, and the nucleic-acid base sequence (SEQ ID No: 1) of the MYOC gene which was predominant in the non-patient group was determined.

(Example 2)

(1) MYOC gene nucleic-acid base sequence polymorphism analysis 1

The blood samples obtained from 88 patients each diagnosed to have an open-angle glaucoma by a medical center were treated in accordance with the procedure of the above-mentioned Example and the nucleic-acid base sequence of the MYOC gene of each patient was analyzed and compared with the nucleic-acid base sequence of the non-patient group.

The results are shown in Table 1. The first row in Table 1 represents the position of the nucleic-acid base sequence of the MYOC gene represented by SEQ ID No: 1, the second row represents the base predominant in the non-patient group in each position, the third row represents the frequency of the variation in each base position in the non-patient group, the fourth row represents the frequency of the variation in each base position in the patient group and the fifth row represents the change in the nucleic-acid base detected as a variation.

As a result, the variation at nucleic-acid base positions of position 324, position 4037 and position 4346 was found at a frequency of about 3% in the non-patient group, while the frequency of the variation in the patient group was 6.8 to 10.2%. In other positions, there was no variation found in the non-patient group, but the variation was found at a frequency of about 1 to 3.4% in the patient group.

(Table 1)

| Base position | 194 | 199 | 324 | 1051 | 1084 | 1627 | 1685 | 1756 | 1853 | 2830 | 3371 | 4037 | 4346 |
|---|---|---|---|---|---|---|---|---|---|---|---|---|---|
| Nucleic-acid base | C | A | G | C | C | T | T | C | G | G | A | G | G |
| Non-patient group | 0.0% | 0.0% | 3.0% | 0.0% | 0.0% | 0.0% | 0.0% | 0.0% | 0.0% | 0.0% | 0.0% | 3.0% | 3.0% |
| Patient group | 3.4% | 1.1% | 6.8% | 2.3% | 3.4% | 3.4% | 2.3% | 2.3% | 2.3% | 1.1% | 1.1% | 10.2% | 10.2% |
| Variation | C→A | A→C | G→A | C→T | C→T | T→C | T→C | C→T | G→C | G→A | A→G | G→A | G→A |

(2) MYOC gene nucleic-acid base sequence polymorphism analysis 2

11 Patients and 2 non-patients having variations in the nucleic-acid bases at position 4037 and position 4346 were also examined for the variations at other positions.

The results are shown in Table 2. The first row of Table 2 represents the nucleic-acid base position, and the second row or later represent the presence or absence of the variation in individual subject.

As a result, the non-patient group exhibited no variations in the base positions other than the positions 4037 and 4346, while the patient group included a subgroup with the variations in the 194, 1084- and position 1627s and a subgroup with the variations in the 1051-, 1685-, 1756- and position 1853s, or a subgroup without variations.

(Table 2)

| Baseposition | 194 | 199 | 324 | 1051 | 1084 | 1627 | 1685 | 1756 | 1853 | 2830 | 3371 | 4037 | 4346 |
|---|---|---|---|---|---|---|---|---|---|---|---|---|---|
| Patient 1 | * | | | | * | * | | | | | | * | * |
| Patient 2 | * | | | | * | * | | | | | | * | * |
| Patient 3 | * | | | | * | * | | | | | | * | * |
| Patient 4 | | | | | | | | | | | | * | * |
| Patient 5 | | | | | | | | | | | | * | * |
| Patient 6 | | | | | | | | | | | | * | * |
| Patient 7 | | | | | | | | | | | | * | * |
| Patient 8 | | | | | | | | | | | | * | * |
| Patient 9 | | | | | | | | | | | | * | * |
| Patient 10 | | | | * | | | * | * | * | | | * | * |
| Patient 11 | | | | * | | | * | * | * | | | * | * |
| Non-patient 1 | | | | | | | | | | | | * | * |
| Non-patient 2 | | | | | | | | | | | | * | * |

(3) Risk judgment

According to the Bayes theory, the risk of developing glaucoma when there is a variation in the MYOC gene sequence is predicted.

The probability of developing glaucoma in future of a in a given subject is judged based on the incidence in a epidemiologically categorized general population, if there is no information beforehand. When this incidence is designated as P(G) and the probability of no development of glaucoma is designated as P(N), then there is a relationship of P(N) =1-P(G).

On the other hand, the case where there is a single or multiple variation in the MYOC gene sequence is designated as M, and the conditional probability that a subject having M will undergo the development of glaucoma is designated as P(G|M). If P(G|M)>P(G), then the probability that the variation M-possessing subject will undergo the development of glaucoma is higher than that in the general population, resulting in a judgment of a high risk subject.

The conditional probability P(G|M) is calculated as follows.

The probability of the possession of M in the glaucoma patient group is designated as P(M|G), while the probability of the possession of M in the non-patient group is designated as P(M|N). Then the P(M|N) is represented by Equation 1 according to the Bayes theory (Figure 2). The value of P(G) may employ a value of 3.56%, which was reported to be the glaucoma patient ratio in the population of 40 years old or older based on the nationwide epidemiological investigation conducted over the period from 1988 to 1989 by Japan Ophthalmologists Association. On the other hand, the probability of the variation in a nucleic-acid base in the MYOC gene in each position in the glaucoma patient and non-patient groups can be extrapolated into Equation 1, P(M|G) value and P(M|N) value, respectively.

(Equation 1)

$$P(G|M) = \frac{P(G) \times P(M|G)}{P(G) \times P(M|G) + P(N) \times P(M|N)}$$

Using the equation shown above to calculate P(G|M) for each variation, the ratio of 194-position=28.1, 199-position=28.1, position 324=2.184, position 1051=28.1, position 1084=28.1, position 1627=28.1, position 1685=28.1, position 1756=28.1, position 1853=28.1, position 2830=28.1, position 3371=28.1, position 4037=3.2, position 4346=3.2, exhibiting a higher value of P(G|M) than P(G), which revealed that the possession of a variance in each of these positions reflects a high risk group of the glaucoma. Accordingly, the detection of the variations in the positions in the gene described above is effective in predicting the risk of developing open-angle glaucoma.

(4) Effective examination for risk judgment

Then, the positions of the variations in the MYOC gene for ensuring an effective risk judgment were analyzed, and the results are shown in Table 2/

Since the probability in the patients having the a variation at position 4037 or position 4346 is about 10%, the risk of developing glaucoma in an individual having a variation in these positions can be judged first. On the other hand, any individual having a variation in these positions has the variations in the both positions. Accordingly, the variation of either one of the position 4037 and position 4346 is detected and employed as an index for judging the glaucoma developmental risk.

Nevertheless, the probabilities of the patients having a variation at position 4037 or position 4346 are about 3% respectively, even in the non-patient group. On the other hand, among the patients having the variation at position 4037 and position 4346, the ones having variations in all of the 194-, 1084- and position 1627s were all the patients (Patient 1, Patient 2, Patient 3). Therefore, if a variation in at least one position of positions 194, 1084 and 1627 is detected in addition to the variation of either one of the position 4037 and position 4346 and used as an index, the pseudo-positive judgment can be avoided in predicting the glaucoma onset.

Similarly, the ones having variations in all of the positions 1051, 1685, 1756 and 1853 were all the patients (Patient 10, Patient 11). Therefore, if the variation in at least one position of the positions 1051, 1685, 1756 and 1853 is detected and used as an index, the pseudo-positive judgment can be avoided in predicting the development of glaucoma.

INDUSTRIAL APPLICABILITY

As described above, the information with regard to the gene variation according to the invention is useful in predicting developing in future of glaucoma. Especially, if the development of open-angle glaucoma can be predicted by detecting the MYOC gene variation using an inventive gene examination method, the prevention of the development at a stage before the development or an early treatment is possible.

## Claims

1. A method for examining a glaucoma related myocilin (MYOC) gene comprising the steps:

   (i) detecting a first variation selected from the group consisting of, in SEQ ID No:1, a variation at position 4037 and a variation at position 4346;
(ii) detecting a second variation selected from the group consisting of, in SEQ ID No:1, a variation at position 194, a variation at position 1051, a variation at position 1084, a variation at position 1627, a variation at position 1685, a variation at position 1756 and a variation at position 1853; and
(iii) determining high risk of development of glaucoma based on the detection of the first and the second variations.

**2.** The method according to claim 1, wherein the said variation at position 4037 is a G-to-A substitution.

**3.** The method according to any of claims 1 or 2, wherein the said variation at position 4346 is a G-to-A substitution.

**4.** The method according to any of claims 1 to 3, wherein the said variation at position 194 is a C-to-A substitution, the said variation at position 1051 is a C-to-T substitution, the said variation at position 1084 is a C-to-T substitution, the said variation at position 1627 is a T-to-C substitution, the said variation at position 1685 is a T-to-C substitution, the said variation at position 1756 is a C-to-T substitution, and the said variation at position 1853 is a G-to-C substitution.

**5.** The method according to any of claims 1 to 4, wherein the said glaucoma is primary open-angle glaucoma or normal tension glaucoma.

## Patentansprüche

**1.** Verfahren zum Untersuchen eines mit dem Glaukom in Beziehung stehenden Myocilin (MYOC) - Gens, umfassend die Schritte:

(i) Nachweisen einer ersten Variation, ausgewählt aus der Gruppe bestehend aus, in SEQ ID NO: 1, einer Variation an Position 4037 und einer Variation an Position 4346;
(ii) Nachweisen einer zweiten Variation, ausgewählt aus der Gruppe bestehend aus, in SEQ ID NO: 1, einer Variation an Position 194, einer Variation an Position 1051, einer Variation an Position 1084, einer Variation an Position 1627, einer Variation an Position 1685, einer Variation an Position 1756 und einer Variation an Position 1853; und
(iii) Bestimmen eines hohen Risikos für die Entwicklung eines Glaukoms, basierend auf dem Nachweis der ersten und der zweiten Variationen.

**2.** Verfahren gemäß Anspruch 1, wobei die Variation an Position 4037 eine G-zu-A-Substitution ist.

**3.** Verfahren gemäß einem der Ansprüche 1 oder 2, wobei die Variation an Position 4346 eine G-zu-A-Substitution ist.

**4.** Verfahren gemäß einem der Ansprüche 1 bis 3, wobei die Variation an Position 194 eine C-zu-A-Substitution, die Variation an Position 1051 eine C-zu-T-Subtitution, die Variation an Position 1084 eine C-zu-T-Substitution, die Variation an Position 1627 eine T-zu-C-Substitution, die Variation an Position 1685 eine T-zu-C-Substitution, die Variation an Position 1756 eine C-zu-T-Substitution und die Variation an Position 1853 eine G-zu-C-Substitution ist.

**5.** Verfahren gemäß einem der Ansprüche 1 bis 4, wobei das Glaukom ein primäres Offenwinkelglaukom oder ein Normaldruckglaukom ist.

## Revendications

**1.** Méthode d'examen d'un glaucome lié au gène de la myociline (MYOC) comprenant les étapes :

(i) de détection d'une première variation sélectionnée dans le groupe consistant, dans la SEQ ID No :1, en une variation à la position 4037 et une variation à la position 4346 ;
(ii) de détection d'une seconde variation sélectionnée parmi le groupe consistant, dans la SEQ ID No :1, en une variation à la position 194, une variation à la position 1051, une variation à la position 1084, une variation à la position 1627, une variation à la position 1685, une variation à la position 1756 et une variation à la position 1853; et
(iii) de détermination du haut risque de développement de glaucome basé sur la détection des premières et secondes variations.

2. Méthode selon la revendication 1, dans laquelle ladite variation à la position 4037 est une substitution de G en A.

3. Méthode selon l'une quelconque des revendications 1 ou 2, dans laquelle ladite variation à la position 4346 est une substitution de G en A.

4. Méthode selon l'une quelconque des revendications 1 à 3, dans laquelle ladite variation à la position 194 est une substitution de C en A, ladite variation à la position 1051 est une substitution de C en T, ladite variation à la position 1084 est une substitution de C en T, ladite variation à la position 1627 est une substitution de T en C, ladite variation à la position 1685 est une substitution de T en C, ladite variation à la position 1756 est une substitution de C en T et ladite variation à la position 1853 est une substitution de G en C.

5. Méthode selon l'une quelconque des revendications 1 à 4, dans laquelle ledit glaucome est un glaucome primaire à angle ouvert ou un glaucome à tension normale.

Figure 1

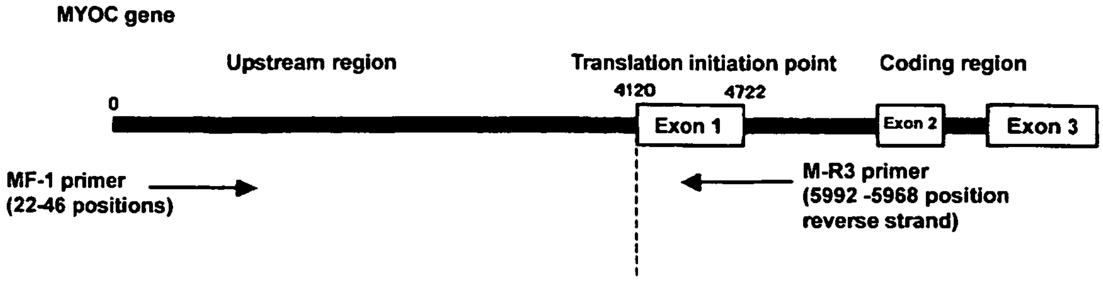


Figure 2

SEQUENCE LISTING

<110> Sysmex Corporation

<120> A check up method for Glaucoma

<130> GP03-1001

<150> JP P2002-093443
<151> 2002-03-29

<160> 27

<170> PatentIn version 3.1

<210> 1
<211> 6000
<212> DNA
<213> Homo sapiens

<400> 1

gctccacagg aagtctcccc actctagact tctgcatcac gatgttacag ccagaagctc 60

cgtgagggtg agggtctgtg tcttacacct acctgtatgc tctacacctg agctcactgc 120

aacctctgcc tcccaggttc aagcaattct cctgtctcag cctcccgcgt agctgggact 180

acaggcgcac gcccggctaa tttttgtatt gttagtagag atggggtttc accatattag 240

cccggctggt cttgaactcc tgacctcagg tgatccaccc acctcagcct cctaaagtgc 300

tgggattaca ggcatgagtc accgcgcccg gccaagggtc agtgtttaat aaggaataac 360

ttgaatggtt tactaaacca acagggaaac agacaaaagc tgtgataatt tcagggattc 420

ttgggatggg gaatggtgcc atgagctgcc tgcctagtcc cagaccactg gtcctcatca 480

ctttcttccc tcatcctcat tttcaggcta agttaccatt ttattcacca tgcttttgtg 540

gtaagcctcc acatcgttac tgaaataaga gtatacataa actagttcca tttggggcca 600

tctgtgtgtg tgtatagggg aggagggcat accccagaga ctccttgaag cccccggcag 660

aggtttcctc tccagctggg ggagccctgc aagcacccgg ggtcctgggt gtcctgagca 720

acctgccagc ccgtgccact ggttgttttg ttatcactct ctagggacct gttgctttct 780

atttctgtgt gactcgttca ttcatccagg cattcattga caatttattg agtacttata 840

tctgccagac accagagaca aaatggtgag caaagcagtc actgccctac cttcgtggag 900

gtgacagttt ctcatggaag acgtgcagaa gaaaattaat agccagccaa cttaaaccca 960

gtgctgaaag aaaggaaata aacaccatct tgaagaattg tgcgcagcat cccttaacaa 1020

ggccacctcc ctagcgcccc ctgctgcctc catcgtgccc ggaggccccc aagcccgagt 1080

cttccaagcc tcctcctcca tcagtcacag cgctgcagct ggcctgcctc gcttcccgtg 1140

aatcgtcctg gtgcatctga gctggagact ccttggctcc aggctccaga aaggaaatgg 1200

agagggaaac tagtctaacg gagaatctgg aggggacagt gtttcctcag agggaaaggg 1260

gcctccacgt ccaggagaat tccaggaggt ggggactgca gggagtgggg acgctggggc 1320

tgagcgggtg ctgaaaggca ggaaggtgaa aagggcaagg ctgaagctgc ccagatgttc 1380

agtgttgttc acggggctgg gagttttccg ttgcttcctg tgagcctttt tatctttttct 1440

ctgcttggag gagaagaagt ctatttcatg aagggatgca gtttcataaa gtcagctgtt 1500

aaaattccag ggtgtgcatg ggttttcctt cacgaaggcc tttatttaat gggaatatag 1560

gaagcgagct catttcctag gccgttaatt cacggaagaa gtgactggag tcttttcttt 1620

catgtcttct gggcaactac tcagccctgt ggtggacttg gcttatgcaa gacggtcgaa 1680

aaccttggaa tcaggagact cggttttctt tctggttctg ccattggttg gctgtgcgac 1740

cgtgggcaag tgtctctcct tccctgggcc atagtcttct ctgctataaa gacccttgca 1800

gctctcgtgt tctgtgaaca cttccctgtg attctctgtg aggggggatg ttgagagggg 1860

aaggaggcag agctggagca gctgagccac aggggaggtg gagggggaca ggaaggcagg 1920

cagaagctgg gtgctccatc agtcctcact gatcacgtca gactccagga ccgagagcca 1980

caatgcttca ggaaagctca atgaacccaa cagccacatt ttccttccct aagcatagac 2040

aatggcattt gccaataacc aaaaagaatg cagagactaa ctggtggtag cttttgcctg 2100

gcattcaaaa actgggccag agcaagtgga aaatgccaga gattgttaaa cttttcaccc 2160

tgaccagcac cccacgcagc tcagcagtga ctgctgacag cacggagtga cctgcagcgc 2220

aggggaggag aagaaaaaga gagggatagt gtatgagcaa gaaagacaga ttcattcaag 2280

ggcagtggga attgaccaca gggattatag tccacgtgat cctgggttct aggaggcagg 2340

gctatattgt gggggaaaa aatcagttca agggaagtcg ggagacctga tttctaatac 2400

tatatttttc ctttacaagc tgagtaattc tgagcaagtc acaaggtagt aactgaggct 2460

gtaagattac ttagtttctc cttattagga actctttttc tctgtggagt tagcagcaca 2520

agggcaatcc cgtttctttt aacaggaaga aaacattcct aagagtaaag ccaaacagat 2580

tcaagcctag gtcttgctga ctatatgatt ggtttttga aaaatcattt cagcgatgtt 2640

tactatctga ttcagaaaat gagactagta ccctttggtc agctgtaaac aaacacccat 2700

ttgtaaatgt ctcaagttca ggcttaactg cagaaccaat caaataagaa tagaatcttt 2760

agagcaaact gtgtttctcc actctggagg tgagtctgcc agggcagttt ggaaatattt 2820

acttcacaag tattgacact gttgttggta ttaacaacat aaagttgctc aaaggcaatc 2880

attatttcaa gtggcttaaa gttacttctg acagttttgg tatatttatt ggctattgcc 2940

atttgctttt tgtttttttct ctttgggttt attaatgtaa agcaggatt attaacctac 3000

agtccagaaa gcctgtgaat ttgaatgagg aaaaaattac atttttgttt ttaccacctt 3060

ctaactaaat ttaacatttt attccattgc gaatagagcc ataaactcaa agtggtaata 3120

acagtacctg tgattttgtc attaccaata gaaatcacag acattttata ctatattaca 3180

gttgttgcag atacgttgta agtgaaatat ttatactcaa aactactttg aaattagacc 3240

tcctgctgga tcttgttttt aacatattaa taaaacatgt ttaaaatttt gatattttga 3300

taatcatatt tcattatcat ttgtttcctt tgtaatctat attttatata tttgaaaaca 3360

tctttctgag aagagttccc cagatttcac caatgaggtt cttggcatgc acacacacag 3420

agtaagaact gatttagagg ctaacattga cattggtgcc tgagatgcaa gactgaaatt 3480

agaaagttct cccaaagata cacagttgtt ttaaagctag ggtgaggggg ggaaatctgc 3540

cgcttctata ggaatgctct ccctggagcc tggtagggtg ctgtccttgt gttctggctg 3600

gctgttattt ttctctgtcc ctgctacgtc ttaaaggact tgtttggatc tccagttcct 3660

agcatagtgc ctggcacagt gcaggttctc aatgagtttg cagagtgaat ggaaatataa 3720

actagaaata tatccttgtt gaaatcagca caccagtagt cctggtgtaa gtgtgtgtac 3780

gtgtgtgtgt gtgtgtgtgt gtgtgtaaaa ccaggtggag atataggaac tattattggg 3840

gtatgggtgc ataaattggg atgttctttt taaaaagaaa ctccaaacag acttctggaa 3900

ggttattttc taagaatctt gctggcagcg tgaaggcaac ccccctgtgc acagccccac 3960

ccagcctcac gtggccacct ctgtcttccc ccatgaaggg ctggctcccc agtatatata 4020

aacctctctg gagctcgggc atgagccagc aaggccaccc atccaggcac ctctcagcac 4080

agcagagctt tccagaggaa gcctcaccaa gcctctgcaa tgaggttctt ctgtgcacgt 4140

tgctgcagct ttgggcctga gatgccagct gtccagctgc tgcttctggc ctgcctggtg 4200

tgggatgtgg gggccaggac agctcagctc aggaaggcca atgaccagag tggccgatgc 4260

cagtatacct tcagtgtggc cagtcccaat gaatccagct gcccagagca gagccaggcc 4320

atgtcagtca tccataactt acagagagac agcagcaccc aacgcttaga cctggaggcc 4380

accaaagctc gactcagctc cctggagagc ctcctccacc aattgacctt ggaccaggct 4440

gccaggcccc aggagaccca ggaggggctg cagagggagc tgggcaccct gaggcgggag 4500

cgggaccagc tggaaaccca aaccagagag ttggagactg cctacagcaa cctcctccga 4560

gacaagtcag ttctggagga agagaagaag cgactaaggc aagaaaatga gaatctggcc 4620

aggaggttgg aaagcagcag ccaggaggta gcaaggctga gaaggggcca gtgtccccag 4680

acccgagaca ctgctcgggc tgtgccacca ggctccagag aaggtaagaa tgcagagtgg 4740

ggggactctg agttcagcag gtgatatggc tcgtagtgac ctgctacagg cgctccaggc 4800

ctccctgcct gccctttctc ctagagactg cacagctagc acaagacaga tgaattaagg 4860

aaagcacagc gatcaccttc aagtattact agtaatttag ctcctgagag cttcatttag 4920

attagtggtt cagagttctt gtgcccctcc atgtcagttt tcacagtcca tagcaaaagg 4980

agaaataaaa ggaccgggtg agatgtgtct gcatatgagc agtagaaagt tgtcaattgt 5040

cccttttgaa aaactatcct tttttgaacc tttgctcaga ttgttatttg taccttttga 5100

tgttaaaatg acctttattt atgaaattac aatagatttg ggaaatgata ataagtggta 5160

agtttttgtt tatttttaaa tgttcttccc tggcaaaata aagagatggc acctctctgt 5220

cagttttctt aatatgttgt tctgaaagtt ttcttactca gtccaatctg agaacctctg 5280

cttttaagtc atcagacaaa ttcttgagat ggctttttct gagaggctct tctgttcatc 5340

ctggtccctt cttgcctaaa ggtgagtctg tgtgtgtgtg gggggggtgc gggggtgagg 5400

tgttggggga ggtcttctta ttagctggga agatggtatt tgtgtcactt tttgtgaaag 5460

tgggctccca aatattccct gttgaggaag tgttctaatc atgaggaaat aagcaagcaa 5520

atccagttgt tggacaatta gtttggactg gtcaaagatg tcagtgccaa ggaagaaaga 5580

aaaaaggggt ggggaagggc ttgttctata ttaaagagac taaagaaatg tgttaaccaa 5640

atgtagtgca tgagtcttga ttggtgtctt catccaaggg ggaaaaaggc tatgaggaac 5700

aggtttggga taactgaggc aatttgactg ctcattatta tgttactgta ttaatgttca 5760

gtttcttggt gagataatga tactgtggtt gcgaaggata aaatctttgt tctatggaga 5820

tacatgctta agtacccagg gtgaggcgtc aggatgtctg caatttgctc tcaaatggtt 5880

gaagaaagac tgcaaatata tagataatga gagaaagaaa ggtaaaacaa ctgtggcaaa 5940

atattaataa ctggtgaatt acaaactggt gaatctaagt atatggggag cttattgtac 6000

<210> 2

<211> 25
<212> DNA
<213> Artificial

<220>
<223> Designed DNA based on MYOC gene

<400> 2
ctctagactt ctgcatcacg atgtt 25

<210> 3
<211> 25
<212> DNA
<213> Artificial

<220>
<223> Designed DNA based on MYOC gene

<400> 3
agctccccat atacttagat tcacc 25

<210> 4
<211> 19
<212> DNA
<213> Artificial

<220>
<223> Desiend DNA based on MYOC gene

<400> 4
actaaaccaa cagggaaac 19

<210> 5
<211> 20
<212> DNA
<213> Artificial

<220>
<223> Designed DNA based on MYOC gene

<400> 5
tggttgtttt gttatcactc 20

<210> 6
<211> 18
<212> DNA
<213> Artificial

<220>
<223> Designed DNA based on MYOC gene

<400> 6
ctcctccatc agtcacag 18

<210> 7
<211> 20
<212> DNA
<213> Artificial

<220>
<223> Deisigned DNA based on MYOC gene

<400> 7
gaagtctatt tcatgaaggg 20

<210> 8
<211> 18
<212> DNA
<213> Artificial

<220>
<223> Designed DNA based on MYOC gene

<400> 8
agctctcgtg ttctgtga 18

<210> 9
<211> 18
<212> DNA
<213> Artificial

<220>
<223> Designed DNA based on MYOC gene

<400> 9
aaacttttca ccctgacc 18

<210> 10
<211> 19
<212> DNA
<213> Artificial

<220>
<223> Designed DNA based on MYOC gene

<400> 10
ttctctgtgg agttagcag 19

<210> 11
<211> 19
<212> DNA
<213> Artificial

<220>
<223> Designed DNA based on MYOC gene

<400> 11
acataaagtt gctcaaagg 19

<210> 12
<211> 20
<212> DNA
<213> Artificial

<220>
<223> Designed DNA based on MYOC gene

<400> 12
ctttgaaatt agacctcctg 20

<210> 13
<211> 20
<212> DNA
<213> Artificial

<220>
<223> Designed DNA based on MYOC gene

<400> 13
gctgttattt ttctctgtcc 20

<210> 14
<211> 18
<212> DNA
<213> Artificial

<220>
<223> Designed DNA based on MYOC gene

<400> 14
ctaagaatct tgctggca 18

<210> 15
<211> 19
<212> DNA
<213> Artificial

<220>
<223> Designed DNA based on MYOC gene

<400> 15
ttcttacctt ctctggagc 19

<210> 16
<211> 19
<212> DNA
<213> Artificial

<220>
<223> Designed DNA based on MYOC gene

<400> 16
ttatggatga ctgacatgg 19

<210> 17
<211> 20
<212> DNA
<213> Artificial

<220>
<223> Designed DNA based on MYOC gene

<400> 17
tttatatata ctggggagcc 20

<210> 18
<211> 18
<212> DNA
<213> Artificial

<220>
<223> Designed DNA based on MYOC gene

<400> 18
ccattcactc tgcaaact 18

<210> 19
<211> 20
<212> DNA
<213> Artificial

<220>
<223> Designed DNA based on MYOC gene

<400> 19
ggaactcttc tcagaaagat 20

<210> 20
<211> 18
<212> DNA
<213> Artificial

<220>
<223> Designed DNA based on MYOC gene

<400> 20
aaaagcaaat ggcaatag 18

<210> 21
<211> 19
<212> DNA
<213> Artificial

<220>
<223> Designed DNA based on MYOC gene

<400> 21
gacctaggct tgaatctgt 19

<210> 22
<211> 19
<212> DNA
<213> Artificial

<220>
<223> Designed DNA based on MYOC gene

<400> 22
tgctcataca ctatccctc 19

<210> 23
<211> 18
<212> DNA

<213> Artificial

<220>
<223> Desigend DNA based on MYOC gene

<400> 23
agtgaggact gatggagc 18

<210> 24
<211> 19
<212> DNA
<213> Artificial

<220>
<223> Designed DNA based on MYOC gene

<400> 24
attcccatta aataaaggc 19

<210> 25
<211> 18
<212> DNA
<213> Artificial

<220>
<223> Designed DNA based on MYOC gene

<400> 25
agtctccagc tcagatgc 18

<210> 26
<211> 18
<212> DNA
<213> Artificial

<220>
<223> Designed DNA based on MYOC gene

<400> 26
attgtcaatg aatgcctg 18

<210> 27
<211> 18
<212> DNA
<213> Artificial

<220>
<223> Designed DNA based on MYOC gene

<400> 27
cagtggtctg ggactagg 18